# EUROPEAN PATENT SPECIFICATION

(11) **EP 4 142 667 B1**
(45) Date of publication and mention of the grant of the patent: **05.06.2024**
(21) Application number: 21725591.8
(22) Date of filing: 22.04.2021
(51) Int. Cl.: A61F 9/007

(54) **VITREORETINAL SURGERY DEVICE**
VORRICHTUNG FÜR DIE VITRORETINALE CHIRURGIE
DISPOSITIF DE CHIRURGIE VITRÉO-RÉTINIENNE

(30) Priority: 27.04.2020 IT 202000009049
(43) Date of publication of application: 08.03.2023
(73) Proprietor: Codenotti, Marco, 20129 Milano (IT); Iuliano, Lorenzo, 20063 Cernusco Sul Naviglio (MI) (IT)
(72) Inventor: Codenotti, Marco, 20129 Milano (IT); Iuliano, Lorenzo, 20063 Cernusco Sul Naviglio (MI) (IT)
(74) Representative: Zermani Biondi Orsi, Umberto
(86) International application number: PCT/IB2021/053317
(87) International publication number: WO 2021/220109

(56) References cited:
- EP-A1- 0 610 247
- EP-B1- 0 610 247
- WO-A1-2018/081295
- US-A1- 2016 015 563

## Description

The present invention relates to a vitreoretinal surgery device.

The present invention finds particular application in the field of eye surgery, and more precisely, in the design and manufacture of surgical instruments adapted to be inserted into the eyeball to perform interventions aimed at the surgical treatment of diseases of the posterior part of the eye, in particular of the vitreous body and retina.

The basic principle of vitreoretinal surgery is based on the introduction of very thin instruments inside the eye, through some small incisions in the sclera, and on the maneuver thereof by the surgeon, who for obvious reasons can thus use a maximum of two instruments in the same operative moment.

During the operating step, the surgeon looks (with a microscope) inside the eye through the pupil, which is dilated with special eye drops, and while illuminating the operating area through a fiber optic torch held in one hand, performs the operations required by means of a special surgical instrument held in the other.

In this regard, however, it should be emphasized that typical vitreoretinal surgery requires the use of much more than one surgical instrument, often replaced and repeatedly used in different operating steps.

The surgeon uses different instruments, often used sequentially and more than once.

For this reason, every time a change in the type of maneuver is required, the surgeon is forced to extract the instrument in use, grip the new instrument (possibly passed by a scrub nurse), and finally reintroduce it into the eye.

This involves many problems, both of a purely temporal nature (longer operating times) and of a medical/surgical nature, since the replacement speed of an instrument is of crucial importance for a successful surgical outcome (example: in the event of hemorrhages or drainage of subretinal fluids) and for the stability of intraocular pressure.

Additionally, since the surgeon must necessarily look away for the introduction of the instrument and then return to the microscope in the operating step, the operator's sight during such long and complex operations suffers considerable fatigue, thereby limiting the number of interventions or the time spent in the operating room. Such an aspect is particularly noted in surgeons with refractive defects or presbyopia (surgeons over 45 years of age).

Therefore, it is the object of the present invention to provide a vitreoretinal surgery device which overcomes the aforementioned drawbacks of the known art.

In particular, it is the object of the present invention to provide a highly versatile and efficient vitreoretinal surgery device.

More precisely, it is the object of the present invention to provide a vitreoretinal surgery device which significantly reduces operating times and facilitates the instrument change for the surgeon.

Said objects are achieved by a vitreoretinal surgery device in accordance with the contents of the subsequent claims.

In particular, said objects are achieved by a vitreoretinal surgery device comprising a main body, a needle or cannula, and a plurality of surgical instruments.

Preferably, the main body (or handpiece) has an elongated shape extending along a longitudinal axis thereof between a first and a second end portion.

The main body is at least partially hollow inside and defines a receiving chamber therein.

The needle or cannula is connected to the main body and departs (or protrudes) away from the first end portion thereof up to a free end thereof. The needle or cannula is also provided with an inner longitudinal cavity in connection with said receiving chamber and sized to receive at least one surgical instrument therein.

According to an aspect of the invention, the device comprises at least a first surgical instrument of elongated shape, at least a second surgical instrument of elongated shape, and a selecting unit associated with said first and second surgical instruments, accommodated at least partially in said receiving chamber and configured to arrange the first surgical instrument in the operating position and the second surgical instrument in the resting position, and vice versa, upon a command given by a user. Therefore, the selecting unit is configured to move alternatively said first or said second surgical instrument from a resting position to an operating position upon a command given by a user.

Advantageously, the device is thereby a multi-instrument device in which there is only one needle or cannula which penetrates the sclera of the eye and in which two (or more) surgical instruments are slidably insertable. This allows the surgeon to keep his gaze on the microscope for the entire duration of the operation, simply replacing the instrument in a simplified manner and without completely removing the needle from the sclera. Such surgical instruments can be for example a vitrectome, a laser endophotocoagulation probe, vitreous forceps (of any type), a back-flush cannula for the exchange of intraocular fluids or gases, endovitreal forceps, spatulas.

Preferably, the first surgical instrument is provided with an active end portion thereof and is reversibly movable between a resting position, in which it is accommodated inside said receiving chamber, and an operating position, in which it is arranged crossing said inner longitudinal cavity with said active end portion protruding from the free end of the needle or cannula.

The second surgical instrument is also elongated in shape and has an active end portion thereof. The second surgical instrument is also reversibly movable between a resting position, in which it is accommodated inside said receiving chamber, and an operating position, in which it is arranged crossing said inner longitudinal cavity with said active end portion protruding from the free end of the needle or cannula. Advantageously, when one of the two instruments is extracted and placed through the needle, the other remains accommodated in the receiving chamber, thus favoring the maneuverability of the device and not hindering the operations by the surgeon.

Therefore, the selecting unit is preferably configured alternatively to:
- move said first surgical instrument from the resting position to the operating position and the second surgical instrument from the operating position to the resting position upon receiving a first signal representative of a selection of said first surgical instrument;
- move said second surgical instrument from the resting position to the operating position and the first surgical instrument from the operating position to the resting position upon receiving a second signal representative of a selection of said second surgical instrument.

In this regard, the selecting unit comprises a control element arranged at least partially outside said main body and operable by the user (surgeon, assistant surgeon, scrub nurse) to generate said first and said second. Preferably, the selecting unit comprises at least one thrust element associated with the first and second surgical instruments and movable parallel to said longitudinal axis between a retracted position and an extracted position, in which the first or second instrument is arranged in the operating position. In this regard, preferably the thrust element is substantially aligned with a central axis of said needle or cannula. Furthermore, the selecting unit preferably comprises at least one drum provided with at least a first seat for receiving said first surgical instrument and at least a second seat for receiving said second surgical instrument. This drum is movable between at least a first position, in which the first receiving seat is aligned with said needle or cannula, and a second position, in which the second receiving seat is aligned with said needle or cannula.

Therefore, the movement of the drum places the instrument required by the surgeon in alignment with the needle or cannula, while the thrust element translates it axially, positioning the active end portion thereof outside the needle or cannula (i.e., in the eye).

Therefore, in the operating position of the first surgical instrument, the drum is preferably positioned so that the first receiving seat is aligned with the needle or cannula and the thrust element associated with the first surgical instrument is arranged in an extracted position, at least partially accommodated inside said first receiving seat.

In the operating position of the second surgical instrument, the drum is positioned so that the second receiving seat is aligned with the needle or cannula and the thrust element associated with the second surgical instrument is arranged in an extracted position, at least partially accommodated inside said second receiving seat.

Operatively, the user (surgeon) thus operates the control element by generating the first signal or the second signal.

Following the generation of the first or second signal, the selecting unit positions the first or second surgical instrument in the operating position (and the second or first surgical instrument in the resting position), respectively.

More in detail, the thrust member is maintained or brought into the retracted position, so that the surgical instrument possibly connected thereto returns to the respective receiving seat.

At this point, the drum moves (e.g., rotates) to bring the receiving seat of the selected surgical instrument at the needle or cannula, or in alignment with the needle or cannula.

The thrust member is then grafted (or comes into contact) with said surgical instrument so as to push it linearly through the needle or cannula up to the operating position.

By means of the use of comprising a driving unit (e.g., pedal board), the surgeon can thus send a driving signal to an actuation member of the first or second surgical instrument, actuating it and thus performing the related maneuver during the operation.

In the case of a vitrectome, for example, the driving signal is representative of the movement of the blade and the suction pressure of the vitreous body.

Otherwise, in the case of a laser probe, the driving signal provides the command to switch on the laser.

Again, if the surgical instrument is a forceps, the driving signal (which can also be mechanical/analogue) is representative of the opening or closing of the arms.

Prior art document US2016015563 discloses a cannula insertion system configured to divide a cannula insertion process into a two-step process but however fails to disclose first and second surgical instruments being reversibly movable between a resting position and an operating position.

These and other features, together with the related advantages, will become more apparent from the following exemplary non-limiting description of a preferred non-exclusive embodiment of a vitreoretinal surgery device as shown in the accompanying drawings, in which:
- figure 1 shows a vitreoretinal surgery device according to the present invention, during use;
- figure 2 diagrammatically shows a vitreoretinal surgery device according to the present invention, in a first embodiment;
- figure 3 shows a diagrammatic side view of a vitreoretinal surgery device according to the present invention, in a second embodiment and with some parts in phantom;
- figure 4 shows a sectional view along section line IV-IV in figure 3;
- figure 5 shows a perspective view of a vitreoretinal surgery device according to the present invention, in a third embodiment;
- figure 6 shows a diagrammatic side view of the vitreoretinal surgery device in figure 5;
- figures 7-8 show portions of the vitreoretinal surgery device in figure 5;
- figures 9-10 diagrammatically show a portion of the vitreoretinal surgery device in figure 5 in two different operating conditions;
- figure 11 schematically shows the same portion of the vitreoretinal surgery device in figure 9 in a further operating condition;
- figure 12 shows the same portion of the device in figure 11 in perspective and with some parts in phantom.

With reference to the accompanying drawings, reference numeral 1 indicates, as a whole, a vitreoretinal surgery device according to the present invention.

Such a device 1 thus belongs to the field of micro-invasive surgical instruments used to operate inside the eye.

The device 1 essentially comprises a main body 2, a needle or cannula 3 and a plurality of surgical instruments 3, 4, 5, 6.

The main body 2 has an elongated shape extending along a longitudinal axis "A" thereof between a first 2a and a second end portion.

The shape of the main body 2 is at least partially substantially cylindrical, possibly with a tapered end having dimensions such as to allow an easy grip by the surgeon or user "S".

Therefore, the main body 2 preferably has at least one grippable portion 2b having a substantially circular section, with a diameter of less than 5 cm.

The main body 2 is preferably at least partially hollow and defines a receiving chamber "C" therein.

The needle or cannula 3 instead has a tubular shape (i.e., hollow inside), with a diameter preferably less than 1 mm (approximately 19 gauge). Therefore, such a needle or cannula 3 has an inner longitudinal cavity extending parallel to the longitudinal axis "A".

Such an inner longitudinal cavity is placed in connection with the receiving chamber "C".

The needle or cannula extends away from the first end portion 2a of the main body 2 to a free end 3a thereof.

Therefore, the needle or cannula 3 extends between a constrained end 3b, fixed to the main body 2 (in particular to the first end 2a), and the free end 3a.

Preferably, the free end 3a is tapered so as to define a tip which facilitates the penetration of the ocular sclera.

According to an aspect of the invention, the device comprises a plurality of surgical instruments 4, 5, 6, 7.

Therefore, the main body 2 has a plurality of seats 11a, 11b, 11c, 11d for receiving said plurality of surgical instruments 4, 5, 6, 7 therein. Advantageously, this allows the surgeon to have a plurality of interchangeable surgical instruments located inside the single main body 2 and selectively extractable in an alternative manner as a function of the operative needs.

Each surgical instrument 4, 5, 6, 7 preferably has an elongated shape and is provided with an active end portion 4a, 5a thereof.

The surgical instruments 4, 5, 6, 7 are of the type used for vitreoretinal surgery and can be selected for example from those in the following list:
- endovitreal forceps of any type (for example: serrated, with internal limiting membrane, asymmetrical);
- vitrectome of any cutting speed or aspect (one-dimensional or two-dimensional);
- back-flush cannula, equally with silicone protection ("soft-tip") or without silicone protection ("blunt");
- laser endophotocoagulation probe;
- endodiathermy coagulator;
- endovitreal forceps (vertical or horizontal);
- spatulas or hooks ("pics").

According to a first embodiment, the device 1 comprises at least a first 4 and a second 5 surgical instrument.

Furthermore, in the preferred embodiment, the presence of a third 6 and/or a fourth 7 surgical instrument is also included.

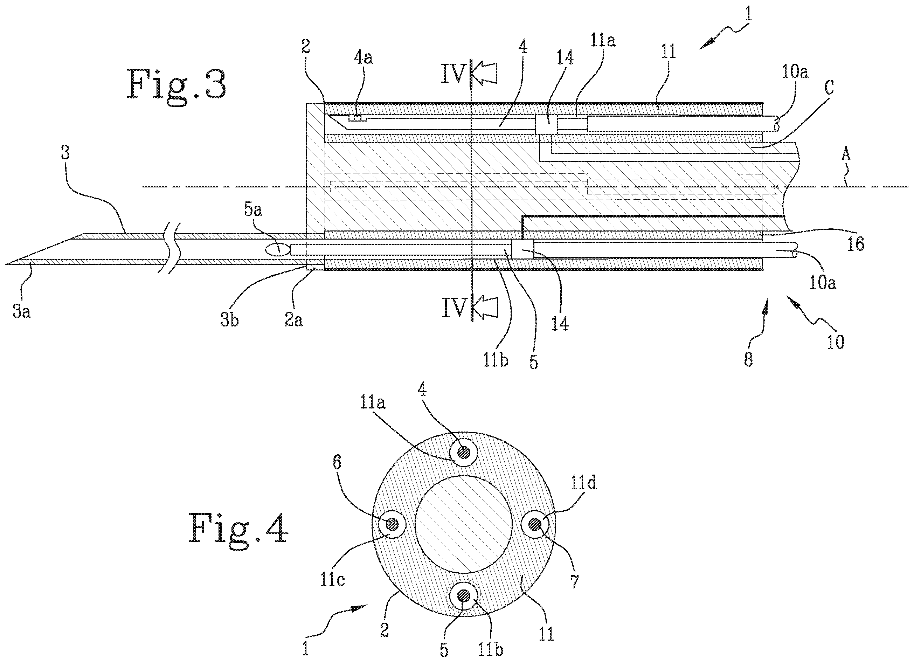

In one of the preferred embodiments, the first surgical instrument 4 is defined by a vitrectome and the second surgical instrument 5 by a forceps. With reference to figures 3 and 4, a laser defining the third surgical instrument 6 and a back-flush cannula defining the fourth surgical instrument 7 are further shown.

Regardless of the nature thereof, all the surgical instruments 4, 5, 6, 7 are reversibly movable between a resting position, in which they are accommodated inside the receiving chamber "C", and an operating position, in which they are arranged crossing the inner longitudinal cavity of the needle or cannula 3, with said active end portion 4a, 5a protruding from the free end 3a.

It should be noted that when a surgical instrument is in the operating position, the other surgical instruments are necessarily in the respective resting position (or in an intermediate position, however not extracted from the needle or cannula).

Therefore, when the first surgical instrument 4 is in the operating position, with the active end portion 4a protruding from the free end 3a, the second surgical instrument 5 is placed in the resting position. Similarly, when the second surgical instrument 5 is in the operating position, with the active end portion 5a protruding from the free end 3a, the first surgical instrument 4 is placed in the resting position.

Furthermore, if present (as in figure 3), the third 6 and/or the fourth 7 surgical instrument are also in said resting position.

Likewise, such third 6 and/or fourth 7 surgical instruments, if present, can be placed in the operating position only with the other instruments in the resting position.

To this end, the device 1 preferably comprises a selecting unit 8 associated with said surgical instruments 4, 5, 6, 7.

The selecting unit 8 is accommodated at least partially in the receiving chamber "C" and is configured to alternatively arrange one of the surgical instruments 4, 5, 6, 7 in the operating position upon a command given by a user "S".

In particular, the selecting unit 8 is configured to move a surgical instrument 4, 5, 6, 7 to the operating position, keeping the others in the resting position.

Therefore, the selecting unit 8 is preferably configured to arrange one of the surgical instruments 4, 5, 6, 7 in the operating position and the remaining (one or more) surgical instruments in the resting position upon receiving a signal (analog or digital) representative of the selection of said surgical instrument.

For example, in embodiments with two surgical instruments, the selecting unit 8 is associated with the first 4 and second 5 surgical instrument and is configured to arrange the first surgical instrument 4 in the operating position and the second surgical instrument 5 in the resting position, or vice versa, upon a command given by the user "S".

More precisely, the selecting unit 8 is thus configured to move the first surgical instrument 4 from the resting position to the operating position and the second surgical instrument 5 from the operating position to the resting position upon receiving a first signal representative of a selection of said first surgical instrument 4.

Furthermore, the selecting unit 8 is configured to move the second surgical instrument 5 from the resting position to the operating position and the first surgical instrument 4 from the operating position to the resting position upon receiving a second signal representative of a selection of said second surgical instrument.

In embodiments with more than two surgical instruments, an example of which is shown in figures 3 and 4, the selecting unit 8 is associated with the first 4, the second 5, the third 6 and/or the fourth 7 surgical instrument and is configured to:
- arrange the first surgical instrument 4 in the operating position and the second 5, third 6 and/or fourth 7 surgical instruments in the resting position, upon receiving a first signal representative of a selection of said first surgical instrument 4;
- arrange the second surgical instrument 5 in the operating position and the first 4, third 6 and/or fourth 7 surgical instruments in the resting position, upon receiving a second signal representative of a selection of said second surgical instrument 4;

- arrange the third surgical instrument 6 in the operating position and the first 4, second 5 and fourth 7 surgical instruments (if present) in the resting position, upon receiving a third signal representative of a selection of said third surgical instrument 6;
- if present, arrange the fourth surgical instrument 7 in the operating position and the first 4, second 5 and third 6 surgical instruments in the resting position, upon receiving a fourth signal representative of a selection of said fourth surgical instrument 7.

In this regard, the selecting unit 8 comprises a control element 9 arranged at least partially outside the main body 2 and operable by the user "S" (surgeon himself, assistant, scrub nurse) to generate the signals representative of the surgical instruments 4, 5, 6, 7 selected.

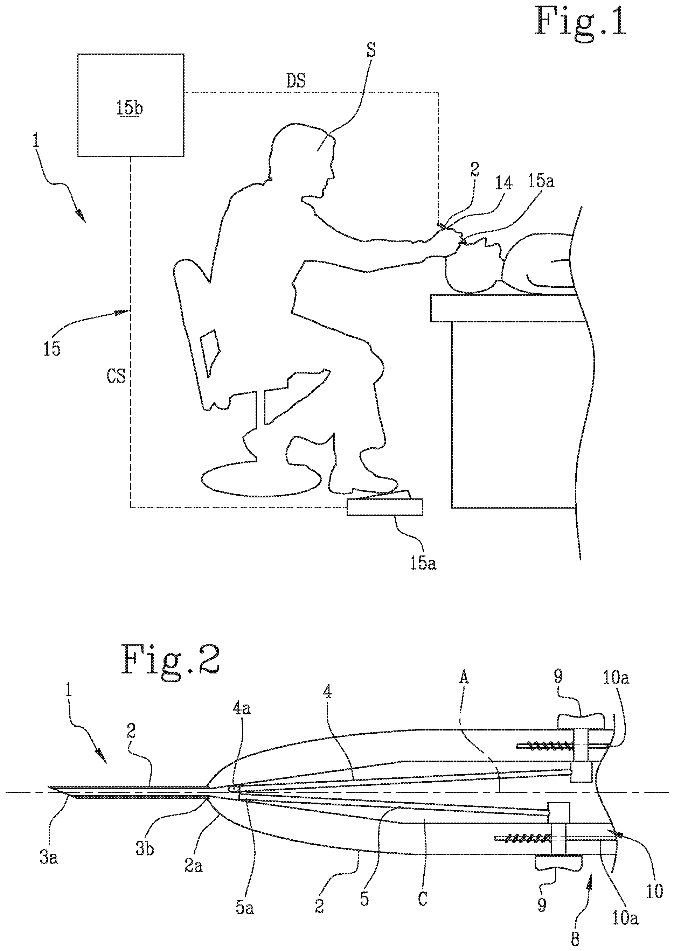

The control element 9 can be defined by a mechanical actuator which can be operated manually by the surgeon (e.g., Figure 2) or by an electronic or electromechanical transducer capable of transforming the surgeon's action into an analog or digital signal.

In the preferred embodiment, the control element 9 is a button configured to generate a related signal representative of the selection of a surgical instrument 4, 5, 6, 7.

Preferably, the selecting unit 8 comprises at least a first actuator 10 partially movable parallel to said longitudinal axis "A" between a retracted position and an extracted position.

Preferably, the first actuator 10 comprises at least one thrust element 10a movable parallel to said longitudinal axis "A" between the retracted position and the extracted position.

In the retracted position, the thrust element 10a is placed in a distal position from the first end portion 2a of the main body 2 and places the surgical instrument 4, 5, 6, 7 in the resting position, or at least allows the placement thereof in such a position.

In the extracted position, the thrust element 10a is instead located near the first end portion 2a of the main body 2.

In such an extracted position, the thrust element 10a is located/configured so as to arrange the surgical instrument 4, 5, 6, 7 associated therewith in the operating position.

It should be noted that the first actuator 10 is configured so as to actively act (i.e., in thrust) on a single surgical instrument, without directly acting on the others.

In this regard, the first actuator 10 can comprise a single thrust element 10a, configured to alternatively couple with only one of the surgical instruments (for example with the first 4 or with the second 5 surgical instrument).

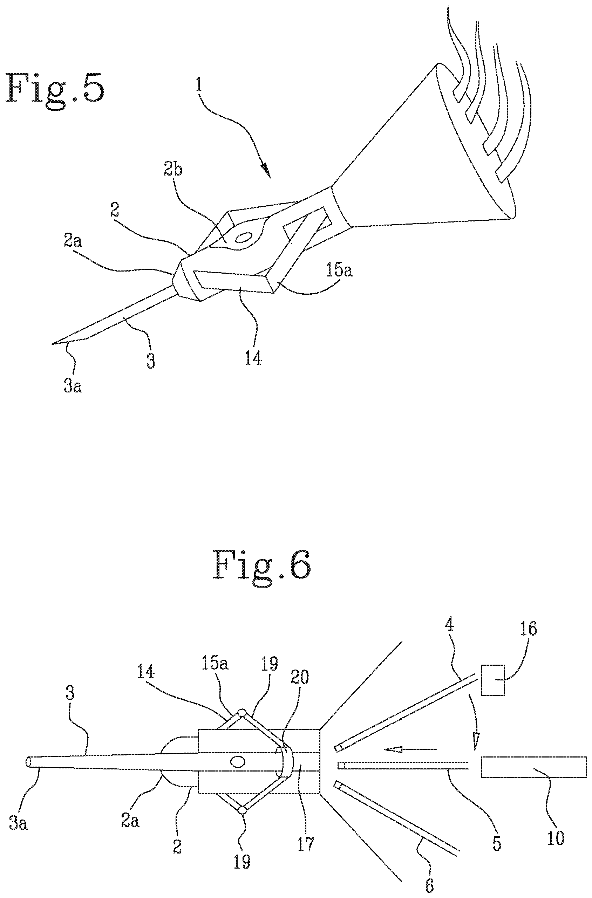

In this embodiment, the thrust element 10a comprises a trigger configured to reversibly couple with each of the surgical instruments 4, 5, 6, 7. Preferably, the thrust element 10a is configured to couple with an end (or end portion) of the surgical instrument opposite the end portion 4a, 5a. With reference to the embodiment in figures 5-8, for example, the device could comprise a single thrust (or traction) element 10a arranged coaxially to said needle or cannula 3 and configured to couple with each of the surgical instruments 4, 5, 6 , 7 as a function of a command given by the user "S".

In such an embodiment, for example, the first actuator 10 comprises a thrust element 10a movable between the retracted position and the extracted position and a gripping unit configured to couple with each instrument previously placed coaxially to the needle or cannula 3. Alternatively, as shown in figures 2, 3, 4, the first actuator 10 comprises a plurality of thrust elements 10a each associated with a respective surgical instrument 4, 5, 6, 7.

With reference to figure 2, the first actuator 10 comprises two thrust elements 10a associated with the first 4 and the second 5 surgical instruments, respectively.

With reference to the embodiment shown in figures 3 and 4, the first actuator 10 instead comprises four thrust elements 10a associated with the first 4, the second 5, the third 6, and the fourth 7 surgical instruments, respectively.

In such embodiments, all the thrust elements 10a are movable between the retracted position and the extracted position in a mutually exclusive manner.

In other words, when a thrust element 10a is in the extracted position, the other thrust elements 10a are in the retracted position.

It should be noted that, preferably, each thrust element 10a is, in at least one operating position thereof, aligned with a central axis of said needle or cannula 3.

In other words, each thrust element 10a is movable from the retracted position to the extracted position (and vice versa) only if aligned with the central axis of said needle or cannula 3.

Advantageously, the movement of the surgical instrument 4, 5, 6, 7 does not thus stress the structure thereof, to the full advantage of the operating precision and reliability of the device 1. It should be noted that, preferably, the first actuator 10 is configured to move each surgical instrument 4, 5, 6, 7 from an intermediate position to the operating position, in which the intermediate position is precisely located along a movement path of the surgical instrument 4, 5, 6, 7 between the resting position and the operating position.

Therefore, the selecting unit 8 preferably comprises a second actuator 16, configured to move each surgical instrument 4, 5, 6, 7 from the resting position to the intermediate position.

In some embodiments, the second actuator 16 of the selecting unit 8 is configured to move all the surgical instruments 4, 5, 6, 7 along a movement path with several stations or steps; at each station or step one of the surgical instruments 4, 5, 6, 7 is placed in the respective intermediate position, aligned with the needle or cannula 3.

An example of such embodiments is shown in figures 3 and 4, in which the selecting unit 8 comprises at least one drum 11 provided with said plurality of receiving seats 11a, 11b, 11c, 11d.

Therefore, the drum 11 has at least a first receiving seat 11a and a second receiving seat 11b for the first 4 and the second surgical instruments, respectively.

In the preferred embodiment, the drum 11 also has a third receiving seat 11c and a fourth receiving seat 11d for the third 6 and the fourth 7 surgical instruments, respectively.

Preferably, the second actuator 16 is configured to move the drum 11 between several positions corresponding to said stations or steps which in number are at least equal to the number of surgical instruments 4, 5, 6, 7; as said, each position is such as to align the respective seat for receiving the surgical instrument with the needle or cannula 3 in order to allow the instrument to be extracted and retracted.

More precisely, the second actuator 16 is configured to receive the signal representative of the surgical instrument 4, 5, 6, 7 selected by the surgeon (generated by the control element 9) and to move the drum 11 upon receiving said signal.

Preferably, each representative signal corresponds to a position of the drum 11 in which the seat for receiving the selected surgical instrument is aligned with the needle or cannula 3.

Therefore, the drum 11 is preferably movable between at least a first position, in which the first receiving seat 11a is aligned with said needle or cannula 3 and at least a second position, in which the second receiving seat 11b is aligned with the needle or cannula 3.

In the preferred embodiment, shown in figures 3 and 4, the drum 11 is movable between at least:
a first position, in which the first receiving seat 11a is aligned with the needle or cannula 3;
a second position, in which the second receiving seat 11b is aligned with the needle or cannula 3;
a third position, in which the third receiving seat 11c is aligned with the needle or cannula 3;
a fourth position, in which the second receiving seat 11d is aligned with the needle or cannula 3.

Preferably, the drum 11 is rotatably accommodated in the main body 2 so as to rotate around said longitudinal axis "A".

Therefore, the positions of the drum 11 are preferably angular positions. Therefore, in the preferred embodiment the second actuator 16 is of the rotary type.

Preferably, the drum 11 has a central portion 12, coaxial to the longitudinal axis "A", and a peripheral portion 13, provided with said receiving seats 11a, 11b, 11c, 11d.

In this regard, it should be noted that the drum 11 can be substantially cylindrical, with a markedly distinct central and peripheral portion, or substantially annular, with a very close central and peripheral portion (see figures 3 and 4).

Advantageously, the drum can thereby easily rotate in a plurality of angular positions, each corresponding to an operating position in which the thrust element 10a can move from the retracted position to the extracted position and, consequently, the corresponding surgical instrument 4, 5, 6, 7 can move from the resting position to the operating position.

Therefore, in the operating position of the first surgical instrument 4, the drum 11 is positioned so that the first receiving seat 11a is aligned with the needle or cannula 3 and the related thrust element 10a is arranged in the extracted position, at least partially accommodated inside said first receiving seat 11a.

In the operating position of the second surgical instrument 5, the drum 11 is positioned so that the second receiving seat 11b is aligned with the needle or cannula 3 and the related thrust element 10a is arranged in the extracted position, at least partially accommodated inside said second receiving seat 11b.

In the operating position of the third surgical instrument 6 (if present), the drum 11 is positioned so that the third receiving seat 11c is aligned with the needle or cannula 3 and the related thrust element 10a is arranged in the extracted position, at least partially accommodated inside said third receiving seat 11c.

In the operating position of the fourth surgical instrument 7 (if present), the drum 11 is positioned so that the fourth receiving seat 11d is aligned with the needle or cannula 3 and the related thrust element 10a is arranged in the extracted position, at least partially accommodated inside said second fourth seat 11d.

The seats for receiving the non-operating surgical instrument(s) (i.e., in the resting position) are instead preferably offset with respect to the needle or cannula 3, or misaligned with respect to said needle or cannula 3. Preferably, the receiving seats of the non-operating surgical instrument(s) are angularly offset with respect to the needle or cannula by an angle equal to the angular offset of each receiving seat with respect to that aligned with the needle or cannula 3.

In the preferred embodiment, the drum 11 is centrally pivoted to the main body 2 to rotate internally thereto around the rotation axis thereof, preferably corresponding to the longitudinal axis.

Preferably, moreover, the receiving seats 11a, 11b, 11c, 11d are angularly equidistant to each other around the rotation axis.

In this regard, the selecting unit 8 comprises an actuation system (not shown)
configured to move the drum 11 to the various positions.

In alternative embodiments, the second actuator 16 is instead configured to individually move each surgical instrument 4, 5, 6, 7 from the resting position to the intermediate position (and vice versa), without moving the others.

In other words, the second actuator 16 can be configured to act on a single surgical instrument 4, 5, 6, 7 at a time, bringing the de-selected instrument from the intermediate position to the resting position in succession and the selected one from the resting position to the intermediate one.

An example of such an embodiment is shown in figures 5 and 6, in which the seats 11a, 11b, 11c, 11d of the surgical instruments 4, 5, 6, 7 are obtained in the main body 2 and are all connected with a single channel 17 (preferably central) aligned with the needle or cannula 3. Such a channel 17 thus corresponds to the intermediate position.

Therefore, in such an embodiment the second actuator 16 is configured to withdraw each surgical instrument 4, 5, 6, 7 from the receiving seat 11a, 11b, 11c, 11d thereof and to position it in said channel 17 (and vice versa); the thrust element 10a is then designed to axially translate the surgical instrument 4, 5, 6, 7 selected into the operating position by moving it from the retracted position to the extracted position.

In the embodiment shown, each surgical instrument 4, 5, 6, 7 in the resting position is arranged in the receiving seat, inclined with respect to the longitudinal axis "A" so that the end portion 4a, 5a is proximal to the axis. The actuation system is thus configured to rotate (i.e., tilt) the selected surgical instrument 4, 5, 6, 7 until it is aligned with the channel 17, in an intermediate position. The thrust element 10a is thus configured to couple with the instrument in the intermediate position and bring it, moving towards the extracted position, to the operating position.

Regardless of the type of selecting unit 8, each surgical instrument 4, 5, 6, 7 preferably comprises an actuation member 14 configured to drive the respective surgical instrument upon receiving a driving signal "DS".

Such an actuation member 14 can be of various nature and location according to the type of surgical instrument; for example, the actuation member 14 can be defined by:
- a pneumatic system of suction and movement of the cutting blade of the vitreous body (for a vitrectome);
- a movement system, which can be mechanical and integrated in the main body 2 or electromechanical/pneumatic arranged partially remotely with respect to the main body 2 (for example for the arms of a forceps or endovitreal forceps);
- a laser energy transfer system (for the laser probe);
- a communication system with the external environment which can be occluded by the surgeon's hand (for the backflush cannula);
- an electrical resistor system, capable of supplying heat (for the diathermocoagulator).

In order to control the actuation members 14, the device 1 comprises a driving system 15 operatively associated with the actuation member of each surgical instrument 4, 5, 6, 7 and configured so as to send said (analog or digital) driving signal "DS" to said actuation member 14 upon a command given by the user "S" (i.e., surgeon).

Such a driving system 15 can be of an electromechanical, pneumatic, or purely mechanical nature, and can be located locally at the main body 2, in a remote position or partially in both positions.

The driving system 15 preferably comprises at least an interface member 15a configured to detect a command by the user "S" and to generate a representative signal of said command "CS".

The interface member 15a can be of various kinds, either integrated in the main body 2 or separated therefrom.

As previously mentioned, for some surgical instruments (e.g., forceps), the interface member 15a is associated with the main body 2 and directly determines the actuation of the respective driving member 14.

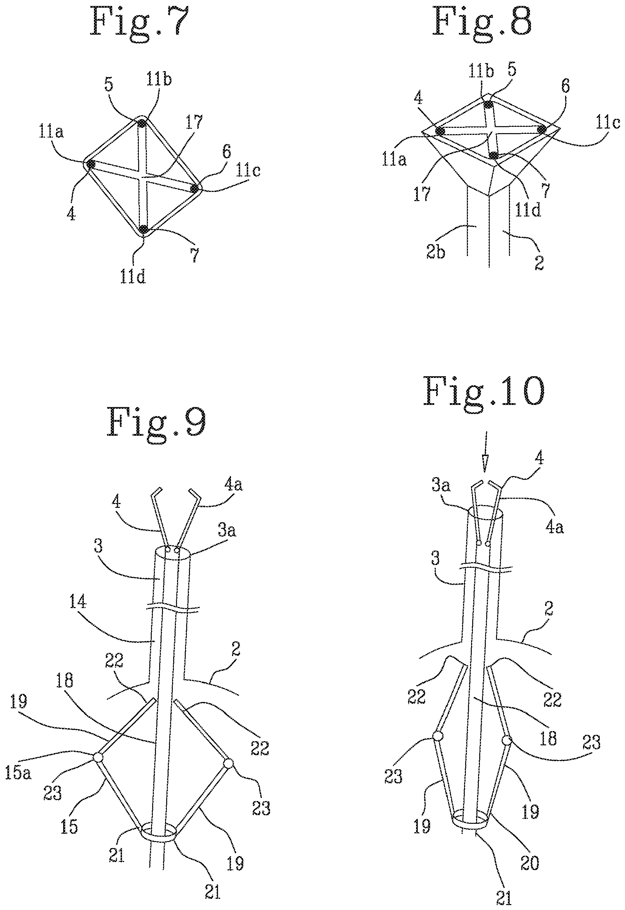

For example, as shown in figures 9-10 for an endovitreal forceps, the driving member 14 comprises a tubular member 18 containing the tool (forceps) and slidingly associated therewith so as to activate it (e.g., when opening or closing) following a relative axial movement between the tubular member and the tool.

In such an embodiment, the interface member 15a is preferably defined by at least one pair of arms 19 (preferably two pairs) which can be connected to the related surgical instrument at least when it is in the intermediate position.

The two arms 19 comprise a fixed hinge 22 and a movable joint 23 such that, following the application of a transversal/orthogonal pressure to the longitudinal axis "A", the angle between the arms 19 increases, translating a ring 20 fitted on the tubular member 18.

The tubular member 18 is in turn provided with an abutment shoulder 21 which, following a pressure from the ring 20, causes a corresponding translation of the tubular member 18 itself and a consequent movement of the tool.

It should be noted that any further tools could be provided with similar tubular members but without abutment shoulders, so that any possible movement of the arms has no effect on the tool.

For further types of surgical instruments, the interface member 15a could be a pedal board provided with a plurality of pedals each configured to generate a special command signal "CS" (see figure 1 in this regard).

In such a configuration, the driving system 15 further comprises a control unit 15b configured to receive said representative signal "CS" and to generate said driving signal "DS".

It should be noted that in some cases the driving system 15 could be hybrid, in part controlled by a remote interface member 15a (e.g., pedal board) and in part by an interface member 15a located on the main body 2.

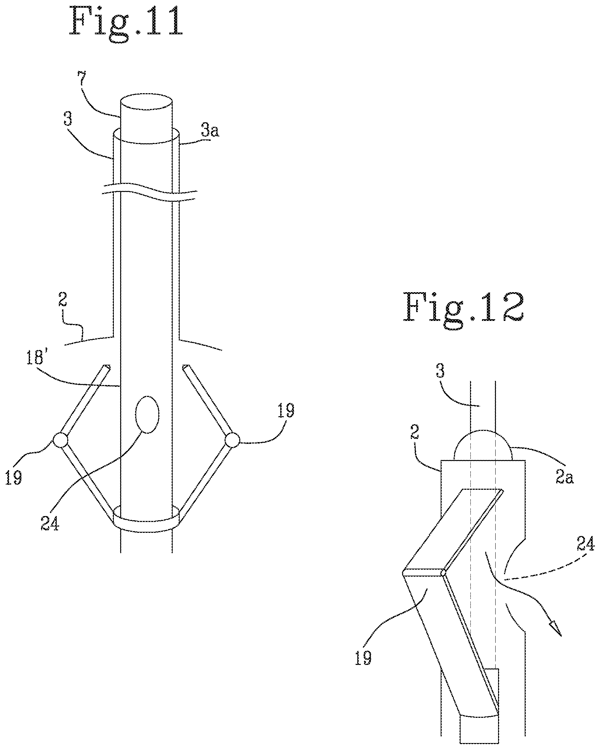

This is the case, for example, of surgical instruments such as the back-flush cannula 7 (figure 11-12), in which the tubular member 18' could lack the abutment described above but provided with an orifice 24 which, in the operating position, is facing and aligned with a corresponding opening 25 made on the main body 2, preferably at the grippable portion 2b, and appropriately sealed thereto (for example by a silicone ring), to allow the user "S" to vary the pressure inside the tubular member 18' by occluding or not occluding the opening 25 (and therefore the orifice 24).

It should be noted that the control element 9 could be integrated in the main body 2 or in the interface member 15a, or still in a further component of the device 1, falling in all cases within the scope of the present invention.

The invention achieves the intended purposes and achieves important advantages.

In fact, the provision of a device which integrates a plurality of interchangeable surgical instruments without removing the needle from the eye greatly facilitates the instrument change operations during surgery, reducing the time of the operation and significantly reducing the fatigue of the surgeon.

## Claims

1. A vitreoretinal surgery device, comprising:
- a main body (2) having an elongated shape extending along a longitudinal axis (A) thereof between a first (2a) and a second end portion and defining a receiving chamber (C) therein;
- a needle or cannula (3) departing away from said first end portion (2a) up to a free end (3a) thereof and provided with an inner longitudinal cavity in connection with said receiving chamber (C);
**characterized in that** it comprises:
- at least a first surgical instrument (4) of elongated shape and provided with an active end portion (4a) thereof; said first surgical instrument (4) being reversibly movable between a resting position, in which it is accommodated inside said receiving chamber (C), and an operating position, in which it is arranged crossing said inner longitudinal cavity with said active end portion (4a) protruding from the free end (3a) of the needle or cannula (3);
- at least a second surgical instrument (5) of elongated shape and provided with an active end portion (5a) thereof; said second surgical instrument (5) being reversibly movable between a resting position, in which it is accommodated inside said receiving chamber (C), and an operating position, in which it is arranged crossing said inner longitudinal cavity with said active end portion (5a) protruding from the free end (3a) of the needle or cannula (3);
- a selecting unit (8) associated with said first and second surgical instruments (5), accommodated at least partially in said receiving chamber (C) and configured to arrange the first surgical instrument (4) in the operating position and the second surgical instrument (5) in the resting position, and vice versa, upon a command given by a user (S).

2. A device according to claim 1, wherein said selecting unit (8) is alternatively configured to:
- move said first surgical instrument (4) from the resting position to the operating position and the second surgical instrument (5) from the operating position to the resting position upon receiving a first signal representative of a selection of said first surgical instrument (4);
- move said second surgical instrument (5) from the resting position to the operating position and the first surgical instrument (4) from the operating position to the resting position upon receiving a second signal representative of a selection of said second surgical instrument (5).

3. A device according to claim 2, wherein said selecting unit (8) comprises a control element (9) arranged at least partially outside said main body (2) and operable by the user (S) to generate said first and said second signals.

4. A device according to any one of the preceding claims, wherein said selecting unit (8) comprises at least a first actuator (10) associated with the first (4) and the second (5) surgical instruments and movable parallel to said longitudinal axis (A) between a retracted position and an extracted position, in which the first (4) or second (5) surgical instrument is arranged in the operating position.

5. A device according to claim 4, wherein said first actuator (10) comprises at least one thrust element (10a) substantially aligned with a central axis of said needle or cannula (3).

6. A device according to claim 4 or 5, wherein the selecting unit (8) comprises a second actuator (16) configured to move each surgical instrument (4, 5, 6, 7) between the resting position and an intermediate position; said first actuator (10) being configured to move each surgical instrument (4, 5, 6, 7) between the intermediate position and the operating position.

7. A device according to claim 6, wherein:
- said selecting unit (8) comprises at least one drum (11) provided with at least a first seat (11a) for receiving said first surgical instrument (4) and at least a second seat (11b) for receiving said second surgical instrument (5);
- said second actuator (16) is configured to move said drum (11) between at least a first position, in which the first receiving seat (11a) is aligned with said needle or cannula (3) and at least a second position, in which the second receiving seat (11b) is aligned with said needle or cannula (3).

8. A device according to claims 4 and 6, wherein:
- in the position of the first surgical instrument (4), the drum (11) is positioned so that the first receiving seat (11a) is aligned with the needle or cannula (3) and the thrust element (10a) associated with the first surgical instrument (4) is arranged in an extracted position, at least partially accommodated inside said first receiving seat (11a);
- in the position of the second surgical instrument (5), the drum (11) is positioned so that the second receiving seat (11b) is aligned with the needle or cannula (3) and the thrust element (10a) associated with the second surgical instrument (5) is arranged in an extracted position, at least partially accommodated inside said second receiving seat (11b).

9. A device according to claim 6, wherein said main body (2) comprises at least a first (11a) and a second (11b) receiving seat for said first (4) and said second surgical instruments (4, 5), respectively; said second actuator (16) being configured to remove each surgical instrument (4, 5) individually from the receiving seat (11a, 11b) thereof and to place it in said intermediate position.

10. A device according to any one of the preceding claims, wherein said first (4) and second (5) surgical instruments are two of the following:
- endovitreal forceps of any type (for example: serrated, with internal limiting membrane, asymmetrical);
- vitrectome of any cutting speed or aspect (one-dimensional or two-dimensional);
- back-flush cannula, equally with silicone protection ("soft-tip") or without silicone protection ("blunt");
- straight-laser endophotocoagulation probe;
- endodiathermy coagulator;
- endovitreal forceps (vertical or horizontal);
- spatulas or hooks ("pics").

11. A device according to any one of the preceding claims, wherein each first (4) or second (5) surgical instrument comprises an actuation member (14) configured to drive the respective surgical instrument upon receiving a driving signal (DS).

12. A device according to claim 11, comprising a driving system (15) operatively associated with each actuation member (14) of the first (4) and second (5) surgical instruments, and configured to send said driving signal (DS) to the respective actuation member (14) upon a command given by the user (S).

13. A device according to claim 12, wherein said driving system (15) comprises at least one interface member (15a) configured to detect a command by the user (S) and to generate a representative signal (CS) of said command.

14. A device according to claim 12 or 13, wherein said driving system (15) comprises a control unit (15b) configured to receive said representative signal (CS) and to generate said driving signal (DS).

15. A device according to any one of the preceding claims, further comprising a third (6) and/or fourth (7) surgical instrument arranged, respectively, in a third (11c) and/or a fourth (11d) receiving seat located in said main body (2).

## Patentansprüche

1. Vorrichtung für die vitreoretinale Chirurgie, umfassend:
- einen Hauptkörper (2) mit einer länglichen Form, der entlang einer Längsachse (A) desselben zwischen einem ersten (2a) und einem zweiten Endabschnitt verläuft und eine Aufnahmekammer (C) darin definiert;
- eine Nadel oder Kanüle (3), die von dem genannten ersten Endabschnitt (2a) ausgehend bis zu einem freien Ende (3a) derselben verläuft und mit einer inneren Längskavität in Verbindung mit der genannten Aufnahmekammer (C) ausgestattet ist;
**dadurch gekennzeichnet, dass** sie Folgendes umfasst:
- mindestens ein erstes chirurgisches Instrument (4) mit länglicher Form, das mit einem aktiven Endabschnitt (4a) desselben ausgestattet ist; wobei das genannte erste chirurgische Instrument (4) reversibel zwischen einer Ruheposition, in der es in der genannten Aufnahmekammer (C) untergebracht ist, und einer Arbeitsposition, in der es die genannte innere Längskavität überquerend angeordnet ist und der genannte aktive Endabschnitt (4a) von dem freien Ende (3a) der Nadel oder Kanüle (3) vorsteht, bewegt werden kann;
- mindestens ein zweites chirurgisches Instrument (5) mit länglicher Form, das mit einem aktiven Endabschnitt (5a) desselben ausgestattet ist; wobei das genannte zweite chirurgische Instrument (5) reversibel zwischen einer Ruheposition, in der es in der genannten Aufnahmekammer (C) untergebracht ist, und einer Arbeitsposition, in der es die genannte innere Längskavität überquerend angeordnet ist und der genannte aktive Endabschnitt (5a) von dem freien Ende (3a) der Nadel oder Kanüle (3) vorsteht, bewegt werden kann;
- eine Auswahleinheit (8), die mit dem genannten ersten und zweiten chirurgischen Instrument (5) verbunden, mindestens teilweise in der genannten Aufnahmekammer (C) untergebracht und darauf ausgelegt ist, auf einen vom Benutzer (S) erteilten Befehl das erste chirurgische Instrument (4) in der Arbeitsposition und das zweite chirurgische Instrument (5) in der Ruheposition und umgekehrt anzuordnen.

2. Vorrichtung nach Anspruch 1, bei der die genannte Auswahleinheit (8) alternativ darauf ausgelegt ist:
- bei Empfang eines ersten, eine Auswahl des genannten ersten chirurgischen Instruments (4) darstellenden Signals das genannte erste chirurgische Instrument (4) aus der Ruheposition in die Arbeitsposition und das zweite chirurgische Instrument (5) aus der Arbeitsposition in die Ruheposition zu bewegen;
- bei Empfang eines zweiten, eine Auswahl des genannten zweiten chirurgischen Instruments (5) darstellenden Signals das genannte zweite chirurgische Instrument (5) aus der Ruheposition in die Arbeitsposition und das erste chirurgische Instrument (4) aus der Arbeitsposition in die Ruheposition zu bewegen.

3. Vorrichtung nach Anspruch 2, wobei die genannte Auswahleinheit (8) ein mindestens teilweise außerhalb des genannten Hauptkörpers (2) angeordnetes Steuerelement (9) umfasst, das vom Benutzer (S) bedient werden kann, um die genannten ersten und zweiten Signale zu erzeugen.

4. Vorrichtung nach einem beliebigen der vorangegangenen Ansprüche, wobei die genannte Auswahleinheit (8) mindestens einen mit dem ersten (4) und dem zweiten (5) chirurgischen Instrument verbundenen ersten Stellantrieb (10) umfasst, der parallel zu der genannten Längsachse (A) zwischen einer zurückgezogenen Position und einer ausgefahrenen Position bewegt werden kann, in der das erste (4) oder zweite (5) chirurgische Instrument in der Arbeitsposition angeordnet ist.

5. Vorrichtung nach Anspruch 4, wobei der genannte erste Stellantrieb (10) mindestens ein im Wesentlichen mit einer mittleren Achse der Nadel oder Kanüle (3) gefluchtetes Schubelement (10a) umfasst.

6. Vorrichtung nach Anspruch 4 oder 5, wobei die Auswahleinheit (8) einen zweiten Stellantrieb (16) umfasst, der darauf ausgelegt ist, jedes chirurgische Instrument (4, 5, 6, 7) zwischen der Ruheposition und einer Zwischenposition zu bewegen; wobei der genannte erste Stellantrieb (10) darauf ausgelegt ist, jedes chirurgische Instrument (4, 5, 6, 7) zwischen der Zwischenposition und der Arbeitsposition zu bewegen.

7. Vorrichtung nach Anspruch 6, wobei:
- die genannte Auswahleinheit (8) mindestens eine mit mindestens einem ersten Sitz (11a) ausgestattete Trommel (11) umfasst, um das genannte erste chirurgische Instrument (4) aufzunehmen, und mindestens einen zweiten Sitz (11b), um das genannte zweite chirurgische Instrument (5) aufzunehmen;
- der genannte zweite Stellantrieb (16) darauf ausgelegt ist, die genannte Trommel (11) zwischen mindestens einer ersten Position, in der der erste Aufnahmesitz (11a) mit der genannten Nadel oder Kanüle (3) gefluchtet ist, und mindestens einer zweiten Position, in der der zweite Aufnahmesitz (11b) mit der genannten Nadel oder Kanüle (3) gefluchtet ist, zu bewegen.

8. Vorrichtung nach Anspruch 4 und 6, wobei:
- in der Position des ersten chirurgischen Instruments (4) die Trommel (11) so positioniert ist, dass der erste Aufnahmesitz (11a) mit der Nadel oder Kanüle (3) gefluchtet ist und das mit dem ersten chirurgischen Instrument (4) verbundene Schubelement (10a) in einer ausgefahrenen Position, mindestens teilweise im Inneren des genannten Aufnahmesitzes (11a), angeordnet ist;
- in der Position des zweiten chirurgischen Instruments (5) die Trommel (11) so positioniert ist, dass der zweite Aufnahmesitz (11b) mit der Nadel oder Kanüle (3) gefluchtet ist und das mit dem zweiten chirurgischen Instrument (5) verbundene Schubelement (10a) in einer ausgefahrenen Position, mindestens teilweise im Inneren des genannten Aufnahmesitzes (11b), angeordnet ist.

9. Vorrichtung nach Anspruch 6, wobei der genannte Hauptkörper (2) mindestens einen ersten (11a) und einen zweiten (11b) Aufnahmesitz jeweils für das genannte erste (4) und das genannte zweite chirurgische Instrument (4, 5) umfasst; wobei der genannte zweite Stellantrieb (16) darauf ausgelegt ist, jedes chirurgische Instrument (4, 5) einzeln aus dem Aufnahmesitz (11a, 11b) desselben zu entfernen und in der genannten Zwischenposition zu platzieren.

10. Vorrichtung nach einem beliebigen der vorangegangenen Ansprüche, wobei das genannte erste (4) und zweite (5) chirurgische Instrument zwei der folgenden Instrumente sind:
- endovitreale Pinzette jeglichen Typs (zum Beispiel: gezahnt, mit interner Begrenzungsmembran, asymmetrisch);
- Vitrektom mit beliebiger Schneidgeschwindigkeit oder beliebigem Aussehen (ein- oder zweidimensional);
- Backflush-Kanüle, gleichermaßen mit Silikonschutz ("weiche Spitze") oder ohne ohne Silikonschutz ("stumpf");
- Endophotokoagulationssonde mit geradem Laser;
- Endodiathermie-Gerinnungsmittel;
- endovitreale Pinzette (vertikal oder horizontal);
- Spatel oder Haken ("pics").

11. Vorrichtung nach einem beliebigen der vorangegangenen Ansprüche, wobei jedes erste (4) oder zweite (5) chirurgische Instrument ein Betätigungsglied (14) umfasst, das darauf ausgelegt ist, das jeweilige chirurgische Instrument beim Erhalt eines Antriebssignals (DS) anzutreiben.

12. Vorrichtung nach Anspruch 11, umfassend ein Antriebssystem (15), das operativ mit jedem Betätigungsglied (14) des ersten (4) und zweiten (5) chirurgischen Instruments verbunden und darauf ausgelegt ist, das genannte Antriebssignal (DS) bei einem vom Benutzer (S) erteilten Befehl an das jeweilige Betätigungsglied (14) zu senden.

13. Vorrichtung nach Anspruch 12, wobei das genannte Antriebssystem (15) mindestens ein Schnittstellenelement (15a) umfasst, das darauf ausgelegt ist, einen vom Benutzer (S) erteilten Befehl zu erkennen und ein repräsentatives Signal (CS) des genannten Befehls zu erzeugen.

14. Vorrichtung nach Anspruch 12 oder 13, wobei das genannte Antriebssystem (15) eine Steuerung (15b) umfasst, die darauf ausgelegt ist, das genannte repräsentative Signal (CS) zu empfangen und das genannte Antriebssignal (DS) zu erzeugen.

15. Vorrichtung nach einem beliebigen der vorangegangenen Ansprüche, die außerdem ein drittes (6) und/oder viertes (7) chirurgisches Instrument umfasst, das jeweils in einem in dem genannten Hauptkörper (2) positionierten dritten (11c) und/oder vierten (11d) Aufnahmesitz angeordnet ist.

## Revendications

1. Dispositif de chirurgie vitréo-rétinienne, comprenant :
- un corps principal (2) ayant une forme allongée s'étendant le long d'un axe longitudinal (A) de celui-ci entre une première (2a) et une deuxième partie d'extrémité et définissant une chambre de réception (C) au sein de celui-ci ;
- une aiguille ou canule (3) s'éloignant de ladite première partie d'extrémité (2a) jusqu'à une extrémité libre (3a) de celle-ci et pourvue d'une cavité longitudinale intérieure en connexion avec ladite chambre de réception (C) ;
**caractérisé en ce qu'**il comprend :
- au moins un premier instrument chirurgical (4) de forme allongée et pourvu d'une partie d'extrémité active (4a) de celui-ci ; ledit premier instrument chirurgical (4) étant mobile de manière réversible entre une position de repos, dans laquelle il est logé à l'intérieur de ladite chambre de réception (C), et une position de fonctionnement, dans laquelle il est agencé de façon à croiser ladite cavité longitudinale intérieure avec ladite partie d'extrémité active (4a) faisant saillie à partir de l'extrémité libre (3a) de l'aiguille ou de la canule (3) ;
- au moins un deuxième instrument chirurgical (5) de forme allongée et pourvu d'une partie d'extrémité active (5a) de celui-ci ; ledit deuxième instrument chirurgical (5) étant mobile de manière réversible entre une position de repos, dans laquelle il est logé à l'intérieur de ladite chambre de réception (C), et une position de fonctionnement, dans laquelle il est agencé de façon à croiser ladite cavité longitudinale intérieure avec ladite partie d'extrémité active (5a) faisant saillie à partir de l'extrémité libre (3a) de l'aiguille ou de la canule (3) ;
- une unité de sélection (8) associée auxdits premier et deuxième instruments chirurgicaux (5), logée au moins partiellement dans ladite chambre de réception (C) et configurée pour agencer le premier instrument chirurgical (4) dans la position de fonctionnement et le deuxième instrument chirurgical (5) dans la position de repos, et vice versa, sur une commande donnée par un utilisateur (S).

2. Dispositif selon la revendication 1, dans lequel ladite unité de sélection (8) est configurée alternativement pour :
- déplacer ledit premier instrument chirurgical (4) de la position de repos à la position de fonctionnement et le deuxième instrument chirurgical (5) de la position de fonctionnement à la position de repos lors de la réception d'un premier signal représentatif d'une sélection dudit premier instrument chirurgical (4) ;
- déplacer ledit deuxième instrument chirurgical (5) de la position de repos à la position de fonctionnement et le premier instrument chirurgical (4) de la position de fonctionnement à la position de repos lors de la réception d'un deuxième signal représentatif d'une sélection dudit deuxième instrument chirurgical (5).

3. Dispositif selon la revendication 2, dans lequel ladite unité de sélection (8) comprend un élément de commande (9) agencé au moins partiellement à l'extérieur dudit corps principal (2) et pouvant être actionné par l'utilisateur (S) pour générer ledit premier et ledit deuxième signal.

4. Dispositif selon l'une quelconque des revendications précédentes, dans lequel ladite unité de sélection (8) comprend au moins un premier actionneur (10) associé aux premier (4) et deuxième (5) instruments chirurgicaux et étant mobile parallèlement audit axe longitudinal (A) entre une position rétractée et une position extraite, dans lequel le premier (4) ou le deuxième (5) instrument chirurgical est agencé dans la position de fonctionnement.

5. Dispositif selon la revendication 4, dans lequel ledit premier actionneur (10) comprend au moins un élément de poussée (10a) sensiblement aligné avec un axe central de ladite aiguille ou canule (3).

6. Dispositif selon la revendication 4 ou 5, dans lequel l'unité de sélection (8) comprend un deuxième actionneur (16) configuré pour déplacer chaque instrument chirurgical (4, 5, 6, 7) entre la position de repos et une position intermédiaire ; ledit premier actionneur (10) étant configuré pour déplacer chaque instrument chirurgical (4, 5, 6, 7) entre la position intermédiaire et la position de fonctionnement.

7. Dispositif selon la revendication 6, dans lequel :
- ladite unité de sélection (8) comprend au moins un tambour (11) pourvu d'au moins un premier siège (11a) pour recevoir ledit premier instrument chirurgical (4) et d'au moins un deuxième siège (11b) pour recevoir ledit deuxième instrument chirurgical (5) ;
- ledit deuxième actionneur (16) est configuré pour déplacer ledit tambour (11) entre au moins une première position, dans laquelle le premier siège de réception (11a) est aligné avec ladite aiguille ou canule (3) et au moins une deuxième position, dans laquelle le deuxième siège de réception (11b) est aligné avec ladite aiguille ou canule (3).

8. Dispositif selon les revendications 4 et 6, dans lequel :
- dans la position du premier instrument chirurgical (4), le tambour (11) est positionné de façon à ce que le premier siège de réception (11a) soit aligné avec l'aiguille ou la canule (3) et l'élément de poussée (10a) associé au premier instrument chirurgical (4) soit agencé dans une position extraite, au moins partiellement logé à l'intérieur dudit premier siège de réception (11a) ;
- dans la position du deuxième instrument chirurgical (5), le tambour (11) est positionné de façon à ce que le deuxième siège de réception (11b) soit aligné avec l'aiguille ou la canule (3) et l'élément de poussée (10a) associé au deuxième instrument chirurgical (5) soit agencé dans une position extraite, au moins partiellement logé à l'intérieur dudit deuxième siège de réception (11b).

9. Dispositif selon la revendication 6, dans lequel ledit corps principal (2) comprend au moins un premier (11a) et un deuxième (11b) siège de réception pour lesdits premier (4) et deuxième instruments chirurgicaux (4, 5), respectivement ; ledit deuxième actionneur (16) étant configuré pour enlever chaque instrument chirurgical (4, 5) individuellement du siège de réception (11a, 11b) de celui-ci et pour le placer dans ladite position intermédiaire.

10. Dispositif selon l'une quelconque des revendications précédentes, dans lequel lesdits premier (4) et deuxième (5) instruments chirurgicaux sont deux parmi les éléments suivants :
- des forceps endo-vitréens d'un type quelconque (par exemple : dentelés, avec une membrane de limitation interne, asymétriques) ;
- vitrectome de vitesse de coupe ou aspect quelconque (unidimensionnel ou bidimensionnel) ;
- canule *back-flush,* également avec protection en silicone (« à embout souple ») ou sans protection en silicone (« émoussé ») ;
- sonde d'endophotocoagulation au laser direct
- agent coagulant d'endodiathermie ;
- des forceps endo-vitréens (verticaux ou horizontaux) ;
- spatules ou crochets (« pics »).

11. Dispositif selon l'une quelconque des revendications précédentes, dans lequel chaque premier (4) ou deuxième (5) instrument chirurgical comprend un élément d'actionnement (14) configuré pour entraîner l'instrument chirurgical respectif lors de la réception d'un signal d'entraînement (DS).

12. Dispositif selon la revendication 11, comprenant un système d'entraînement (15) associé fonctionnellement à chaque élément d'actionnement (14) des premier (4) et deuxième (5) instruments chirurgicaux, et configuré pour envoyer ledit signal d'entraînement (DS) à l'élément d'actionnement (14) respectif sur une commande donnée par l'utilisateur (S) .

13. Dispositif selon la revendication 12, dans lequel ledit système d'entraînement (15) comprend au moins un élément d'interface (15a) configuré pour détecter une commande de la part de l'utilisateur (S) et pour générer un signal représentatif (CS) de ladite commande.

14. Dispositif selon la revendication 12 ou 13, dans lequel ledit système d'entraînement (15) comprend une unité de commande (15b) configurée pour recevoir ledit signal représentatif (CS) et pour générer ledit signal d'entraînement (DS).

15. Dispositif selon l'une quelconque des revendications précédentes, comprenant en outre un troisième (6) et/ou un quatrième (7) instrument chirurgical agencé, respectivement, dans un troisième (11c) et/ou un quatrième (11d) siège de réception situé dans ledit corps principal (2).
